# EUROPEAN PATENT SPECIFICATION

(11) **EP 1 712 915 B1**
(45) Date of publication and mention of the grant of the patent: **24.08.2011**
(21) Application number: 05709624.0
(22) Date of filing: 02.02.2005
(51) Int. Cl.: G01N 33/553, G01N 33/543

(54) **METHOD OF DETECTING ANALYTE WITH USE OF MAGNETIC BEAD**
VERFAHREN ZUM NACHWEIS VON ANALYT UNTER VERWENDUNG VON MAGNETKÜGELCHEN
PROCEDE DE DETECTION D'UN ANALYTE EN UTLISANT UN CORDON MAGNETIQUE

(30) Priority: 03.02.2004 JP 2004026237
(43) Date of publication of application: 18.10.2006
(73) Proprietor: Asahi Kasei Kabushiki Kaisha, Osaka-shi, Osaka 530-8205 (JP)
(72) Inventor: FUJIMURA, Mariko, Nagoya-shi, Aichi 465-0092 (JP); MATSUYAMA, Kenji, Shizuoka-shi, Shizuoka 420-0868 (JP); WATANABE, Katsuya, Shizuoka-shi, Shizuoka 424-0872 (JP)
(74) Representative: Forstmeyer, Dietmar
(86) International application number: PCT/JP2005/001504
(87) International publication number: WO 2005/075997

(56) References cited:
- EP-A- 1 617 220
- WO-A-97/46582
- DE-A1- 10 046 508
- JP-A- 8 043 391
- JP-A- 11 108 926
- JP-A- 2000 187 035
- JP-A- 2001 330 614
- JP-A- 2003 509 034
- JP-A- 2004 331 953

## Description

### Technical Field

The present invention relates to a method of detecting and measuring the presence or the amount of an analyte in a sample easily with high sensitivity. More specifically, the present invention relates to a method of detecting the presence and determining quantities of an analyte using a labeled specific binding material in which a substance (e.g., antibody) capable of specifically binding to an analyte (e.g., antigen) is coupled to magnetic beads via a spacer which is polyalkylene glycol, utilizing the specific reaction between the labeled specific binding material and the analyte by detecting a magnetic signal emitted from the labeled specific binding material by a magnetic sensor. Accordingly, the present invention is useful in the field of life science, in particular, medicine and clinical examination.

### Background Art

Typical examples of methods of detecting an analyte as in the present invention include immunoassay (also referred to as imlnuno-quantitative determination) using an antigen as an analyte. It is conventionally known that an antigen is detected based on the data obtained from a labeling agent coupled to an antibody in immunoassay. Further, methods in which magnetic beads are used as a labeling agent have been conventionally known (Patent Document 1). However, in the method disclosed in Patent Document 1, in the case of an antibody (secondary antibody) coupled to magnetic beads having a diameter of several nanometers to several microns, which are a labeling agent, the magnetic beads are larger than the antibody, and the large specific gravity of the magnetic beads poses a problem that movement and diffusion of the secondary antibody are extremely low, the rate of the antigen-antibody reaction is decreased and thus the detection sensitivity cannot be maintained. Moreover, as magnetic beads are used for detecting magnetic signals, practically the larger the magnetic beads, the more advantageous in terms of the detection sensitivity. Accordingly, diameters a few to 10 times larger than that of gold colloid, latex or polystyrene beads used for usual immunochromatography are employed. This is also a major problem in using magnetic beads as a labeling agent.

On the other hand, there is another mode of immunoassay in which an antigen is bound to an antibody (primary antibody) coupled to magnetic beads which are not used as a labeling agent, and the antigen is further bound to another antibody (secondary antibody) coupled to a fluorescent material or an enzyme which is a labeling agent to form a sandwich structure composed of (primary antibody)-(antigen)-(secondary antibody), and the structured body is selectively agglomerated utilizing characteristics of the magnetic beads which the primary antibody contains (BF, binding free, separation), thereby detecting the antigen with the labeling agent such as a fluorescent material or an enzyme (Patent Document 2).
A still another mode is to use a material obtained by further attaching an antigen to a conjugate in which an antibody is coupled to magnetic beads via a spacer for magnetic separation/magnetic transport to recover the antigen utilizing characteristics of the magnetic beads (Patent Document 3).
However, since both the above immunoassay using a sandwich structure and the magnetic separation/magnetic transport do not use magnetic beads as a labeling agent, such publications do not specify the details, for example, the size, of magnetic beads. Patent Document 2 specifically discloses that magnetic beads having a diameter of about 0.01 µm are used. Use of magnetic beads having such a size as a labeling agent poses a problem that detection of analytes is difficult because the magnetic beads are small and the signal obtained is small.

In short, labeled specific binding materials do not exist at present in which magnetic beads capable of generating magnetic signals sufficient for detection are used as a labeling agent, in which a substance capable of specifically binding to an analyte is provided on the magnetic beads, and which has high reaction efficiency with the analyte.

Patent Document 1: Japanese National Publication of International Patent Application No. 2001-524675
Patent Document 2: Japanese Patent Laid-Open No. 4-323560
Patent Document 3: Japanese Patent Laid-Open No. 2002-131320

### Disclosure of the Invention

### Problems to be Solved by the Invention

An object of the present invention is to provide a labeled specific binding material having high reaction efficiency with an analyte and capable of generating magnetic signals sufficient for detection in a method of detecting an analyte, comprising detecting a signal from magnetic beads in a conjugate obtained by attaching an analyte to a material (labeled specific binding material) which is labeled with magnetic beads and which specifically binds to the analyte, thereby detecting the analyte, and a method of detecting an analyte using the same.

### Means for Solving the Problem

The present inventors have conducted intensive studies to solve the above problems and as a result, succeeded in detecting an analyte with high accuracy using a labeled specific binding material in which magnetic beads having a specific size is used as a labeling agent and a substance capable of specifically binding to an analyte is coupled to the magnetic beads via a spacer having a specific length.

Accordingly, the present invention relates to:
1. a labeled specific binding material comprising a substance capable of specifically binding to an analyte, a spacer and magnetic beads having a particle size of 0.5 to 10 µm, wherein the specific binding substance is coupled to the magnetic beads via the spacer and
   , wherein the spacer is polyalkylene glycol having 50
   to 500 repeat units;
2. the labeled specific binding material according to 1, wherein the polyalkylene glycol is polyethylene glycol;
3. the labeled specific binding material according to any one of 1. to 2., wherein the spacer is bonded to the magnetic beads through an avidin/biotin complex;
4. the labeled specific binding material according to any one of 1. to 3, wherein the analyte is an antigen and the substance capable of specifically binding to the analyte is an antibody;
5. a kit for detecting an analyte, comprising a labeled specific binding material according to any one of 1. to 4; and
6. An in vitro method of detecting an analyte, comprising binding the analyte to the labeled specific binding material according to any one of 1. to 4. to form a conjugate, and detecting a magnetic signal from the conjugate to detect the analyte.
7. In vitro use of a labelled specific binding material according to any one of claims 1 to 4 for detecting an analyte.
8. Use of a labelled specific binding material according to any one of claims 1 to 4 for provision of a kit for detecting an analyte.

### Advantages of the Invention

The present invention improves the reaction rate between a labeled specific binding material such as a magnetic bead labeled secondary antibody and an analyte, and also achieves high sensitivity magnetic sensor measurement based on high sensitivity magnetic sensing.
The technique of detecting an analyte according to the present invention can be applied to qualification and determination of various analytes such as antigens and ligands. In particular, the present invention can be suitably applied to the field of medical diagnosis and test agents including tests on antigens contained in blood, various body fluids and wipe liquids using immunoassay.

### Brief Description of the Drawings

Figure 1 is a schematic view illustrating an embodiment of a signal detection system (magnetic measuring instrument) according to the present invention; and
Figure 2 is a view illustrating a processing method in the signal detection system (signal processor) in Examples 2 and 4 of the present invention.

### Description of symbols

101 two dimensional rotation center
102 magnetic field generator
103 magnetic sensor
104 sample base
106 stationary table
107 rotary table
1030 driving function
1010 drive rotation center
1020 drive transfer function
201 amplifier
202 position detection means
203 analogue-digital converter
204 drive control function
205 central processing unit
206 communication means
207 power
208 display means
209 storage medium
210 battery

### Best Mode for Carrying out the Invention

The present invention will be described in detail below.

First, the analyte in the present invention means one substance of a pair of specific binding substances, such as a ligand for a receptor and an antigen for an antibody, which is particularly difficult to be directly detected in the fields of medicine and clinical examination. Examples thereof include the above-described ligands, antigens and complementary DNA.

In the following description, an embodiment using an antigen as an analyte is described, but the analyte in the present invention is not limited to antigens. In the following description, an antigen corresponds to an analyte, an antibody corresponds to a substance specifically binds to an analyte, and a labeled secondary antibody corresponds to a labeled specific binding material.

Antigens and antibodies may be those involved in usual antigen-antibody reaction. Examples thereof include combination of a C-polysaccharide antigen and a purified fraction of an anti-C-polysaccharide antibody (rabbit polyclonal antibody available from Statens Serum Institut, Denmark) through a protein G column, or a ribosomal protein L7/L12 antibody for bacteria to be detected and a corresponding ribosomal protein L7/L12 antigen of the bacteria disclosed in European Patent No. 1104772. Specific examples thereof include combination of anti-Mycoplasma pneumoniae antibody AMMP-1 and ribosomal protein L7/L12 of Mycoplasma pneumoniae, combination of anti-Mycoplasma pneumoniae antibody AMMP-2 to 5 derived from a sibling strain MPRB-2 to 5 of a producing strain MPRB-1 of anti-Mycoplasma pneumoniae antibody AMMP-1 and ribosomal protein L7/L12 of Mycoplasma pneumoniae, combination of anti-Haemophilus influenzae antibody HIRB-2 and ribosomal protein L7/L12 of Haemophilus influenzae, combination of anti-Streptococcus pneumoniae antibody AMSP-2 and ribosomal protein L7/L12 of Streptococcus pneumoniae, and combination of anti-Chlamydia pneumoniae antibody AMCP-1 and ribosomal protein L7/L12 of Chlamydia pneumoniae disclosed in the above European Patent. Combination of an antibody and an antigen applicable to the present invention is not limited to these combinations.
Of the above anti-Mycoplasma pneumoniae antibodies AMMP-1 to 5, AMMP-1 is preferred because it binds to only Mycoplasma pneumoniae one on one with high reactivity.

A characteristic of the present invention resides in the structure of a secondary antibody (labeled secondary antibody) in which an antibody which specifically binds to an antigen is coupled to a labeling agent. Accordingly, labeled secondary antibodies which may be used in the present invention are now described.
Labeled secondary antibodies which may be used in the present invention characteristically use magnetic beads as a labeling agent.
Magnetic beads which may be used in the present invention are particles magnetized at least while a magnetic field is externally applied. Examples of such magnetic beads include particles obtained by forming a magnetic body alone into particles, particles composed of a magnetic body as a core whose surface is covered with a polymer material such as polystyrene, silica gel, gelatin or polyacrylamide, particles composed of a polymer material such as polystyrene, silica gel, gelatin or polyacrylamide as a core whose surface is covered with a magnetic body, and particles obtained by encapsulating a magnetic body into a closed vesicular materials such as erythrocyte, liposome or microcapsules. In the present invention, particles composed of a magnetic body as a core whose surface is covered with a polymer material such as polystyrene, silica gel, gelatin or polyacrylamide, to which antibodies or other substances can be easily coupled, are preferred, because it is necessary to form a labeled secondary antibody by coupling an antibody or the like to the surface of magnetic beads as described later.

Examples of magnetic bodies described above include ferromagnetic metals such as iron, cobalt and nickel, alloys containing the same, non-magnetic bodies containing the above ferromagnetic metal or alloy containing the same, and the above ferromagnetic metal or alloy containing the ferromagnetic metal, which contain a non-magnetic body.
The magnetic beads which may be used in the present invention are particularly preferably those generally called a superparamagnetic body, which has a characteristic of being magnetized while a magnet is externally applied and immediately demagnetized when the application of the magnet is discontinued.
Examples of magnetic beads having properties described above include, but not limited to, Dynabeads M-450, M-270, M-280 (Dynabeads is a registered trademark) and Dynabeads Myone (registered trademark) available from Dynal Biotech ASA, Norway, and Sera-mag (registered trademark) available from Seradyn Inc., USA.
When magnetic beads have a small particle size, the absolute amount of the secondary antibody magnetic body bound to an antigen is decreased, and therefore sufficient sensitivity cannot be obtained in a magnetic sensor. Accordingly, the magnetic beads in the present invention have a particle size of 0.1 to 10 µm, preferably 0.5 to 10 µm. The shape of particles is not particularly limited, and may be spherical or polyhedral.

The labeled secondary antibody which may be used in the present invention has a structure in which an antibody is coupled to magnetic beads which is a labeling agent. To produce an effect of achieving specific binding between an antigen and an antibody with high efficiency, preferably magnetic beads and an antibody are coupled via a spacer.
The spacer which may be used in the present invention may be those which are hydrophilic. Examples thereof include polyalkylene glycol, sugar chains and phospholipids. Of these, polyalkylene glycol whose molecules are less likely to be entangled with each other as spacer is preferred.
Examples of polyalkylene glycol which may be used in the present invention include various equivalent compounds such as polypropylene glycol and polyethylene glycol. Of these, polyethylene glycol is particularly preferably used.

While spacers of various lengths may be used in the present invention, spacers have a specific length of preferably 10 Å to 2000 Å, more preferably 200 Å to 2000 Å in order to produce a higher effect. Such a length can be obtained by, for example, in the case of polyalkylene glycol (hereinafter may be abbreviated as PALG), a structure in which 2 to 500, particularly 50 to 500, PALG monomers are repeated. When polyethylene glycol (hereinafter PEG) is used as polyalkylene glycol, the length can be obtained when polyethylene glycol has a weight average molecular weight of 2200 to 22000, preferably approximately 3000 within 2500 to 4000.
In the present invention, while the magnetic beads which are a labeling agent may have a size and the spacer may have a length satisfying the above conditions, a higher effect is produced when the size (R) of the magnetic beads and the length (L) of the spacer satisfy a relation R/L of 0.5 to 10000, more preferably 2.5 to 500.

The spacer in the present invention is a linear hydrophilic compound located between magnetic beads and an antibody coupled thereto. The presence of such a spacer allows an antibody to move freely in a reaction mixture, increasing the reactivity between the antibody which is a labeled secondary antibody and an antigen, and as a result, the antigen-antibody reaction rate of the labeled secondary antibody labeled with magnetic beads is probably significantly increased. Accordingly, even when the magnetic beads have a large size and a high specific gravity, the antigen-antibody reaction rate between an antigen and a labeled secondary antibody is increased with high detection sensitivity. Thus, if improvement in magnetic properties allows magnetic beads to have a smaller diameter in the future, the reaction rate between an antigen and an antibody will be further increased along with increased freedom in movement of an antibody due to such a small diameter.

In the present invention, when magnetic beads of a magnetic body as a core whose surface is covered with a polymer material such as polystyrene, silica gel, gelatin or polyacrylamide are used for a labeled secondary antibody, an approach of coupling magnetic beads to a spacer through a covalent bond utilizing a functional group such as a COOH group or a NH group on the surface of the magnetic beads may be used. It is desired, however, that a labeled secondary antibody is formed using a biotinylated spacer and avidinylated magnetic beads through an avidin-biotin complex.

An example of methods of preparing a labeled secondary antibody obtained by coupling a spacer to magnetic beads through an avidin-biotin complex using PEG as a spacer is described below.
First, a PEG chain and biotin are introduced into an antibody using PEG whose one terminal is biotin and the other is a functional group such as -NHS or maleimide.
In a buffer such as phosphate buffered saline (hereinafter PBS) or tetraborate, 3 to 10 mole equivalents of a Biotin-PEG-CO₂-NHS reagent (MW3400 available from Shearwater Polymers Inc., USA) dissolved in distilled water is added to 0.1 mg to 10 mg (6.7×10⁻⁷ mmol to 6.7×10⁻⁵ mmol) of an antibody. The mixture is allowed to react at 4°C to room temperature for 2 to 12 hours. The reaction mixture is purified by centrifugal ultrafiltration or gel filtration to give a PEG-biotinylated antibody solution.
The biotinylation per molecule of the resulting PEG-biotinylated antibody solution was determined using a HABA regent (available from Pierce Biotechnology, Inc., USA). The Biotinylation degree is observed to be 1 to 10 biotin/per antibody molecule.

Secondly, 0.1 mg to 10 mg of magnetic beads, i.e., Dynabeads M-270 streptavidin (available from Dynal Biotech ASA, Norway, diameter 2.8 µm) are prepared, and the above PEG-biotinylated antibody is added thereto so that the composition ratio of the magnetic beads to the PEG-biotinylated antibody is PEG-biotinylated antibody/magnetic beads =1/1 to 1/100 in weight ratio. The mixture is allowed to react with stirring at 4°C to room temperature for 1 to 12 hours. Only the magnetic bead labeled secondary antibody is recovered from the Magnetic bead labeled secondary antibody solution obtained in the reaction using a magnet, washed with PBS several times, and the bead concentration of the prepared magnetic bead labeled secondary antibody is finally adjusted to 0.01 % to 1 % with a 1 % BSA/PBS solution (BSA: bovine serum albumin).
An example of methods of preparing a labeled secondary antibody which may be used in the present invention has been described above.

In the present invention, the presence of an antigen and amounts thereof can be directly detected from a conjugate obtained by antigen-antibody reaction between a labeled secondary antibody and an antigen. However, to achieve higher detection accuracy, it is preferred that the antigen in the conjugate undergoes antigen-antibody reaction with an antibody in a primary antibody immobilized on a detection area to form a sandwich structure composed of (labeled secondary antibody)-(antigen)-(immobilized primary antibody) in the detection area, thereby detecting magnetic signals emitted from magnetic beads in the structure immobilized on the detection area to detect an antigen.

Accordingly, the primary antibody immobilized on a detection area is now described.
The antibody used for the primary antibody may be the same as or different from the antibody used for the secondary antibody, but the primary antibody must specifically bind to an antigen which is an analyte. The primary antibody can be immobilized using various materials such as polystyrene, polydimethylsiloxane-coat silicon, nitrocellulose and glass fiber generally used as an adsorbing substrate for a primary antibody in immunoassay. Alternatively, by a covalent binding method utilizing a NH₂ residue, a COOH residue or a SH group in a primary antibody, the primary antibody can be fixed to various materials, e.g., glass substrates, polystyrene, polydimethylsiloxane-coat silicon, nitrocellulose and glass fiber which have a functional group on the surface through a covalent bond to form a detection area in the present invention.

An example of methods of preparing a detection area on which a primary antibody is immobilized is described below.
1 to 50 µl of an anti-C-polysacchride antibody (rabbit polyclonal antibody available from Statens Serum Institut, Denmark) dissolved in an appropriate buffer such as a sodium phosphate buffer in a concentration of 1 µg/ml to 50 µg/ml is spotted on a polystyrene substrate. The antibody is allowed to react at 4°C to room temperature for 30 minutes to 24 hours in a humidified box. The surface of the substrate is washed with distilled water and then 1 µl to 50 µl of a 1% BSA/PBS solution is spotted thereon and reaction is performed at 4°C to room temperature for 30 minutes to 24 hours in a humidified box. The surface of the substrate is washed with distilled water and dried to give a primary antibody immobilized substrate.

The method of detection of an antigen using the above-described labeled secondary antibody and the primary antibody immobilized on a detection area is now described.
At first, a method of preparing a sample used in the detection is described.
First, a buffer containing an antigen, such as PBS, is spotted on a primary antibody immobilized on a detection area, and with leaving at 4°C to room temperature for 5 minutes to 1 hour, the antigen and the primary antibody are allowed to react and the antigen is bound to the primary antibody.
Subsequently, 1 to 50 µl of a reagent containing a magnetic bead labeled secondary antibody in a bead concentration of 0.01 % to 1 % is dropped on the detection area, and with leaving at 4°C to room temperature for 5 minutes to 1 hour, the antigen bound to the primary antibody is allowed to react with the labeled secondary antibody. Then, unreacted secondary antibody is washed away with distilled water or the like to give a (labeled secondary antibody)-(antigen)-(immobilized primary antibody) sandwich structure.
In the present invention, detection is performed using a sample having such a sandwich structure immobilized on a detection area prepared as described above.

The above sample can also be prepared using a detection kit in which a series of steps shown below can be performed.
Specifically, the detection kit has a labeled secondary antibody carrying area where a labeled secondary antibody is previously carried on a carrier such as glass fiber, non-woven fabric or nitrocellulose and a detection area on which a primary antibody is immobilized. First, settings are made so that a buffer containing an antigen, such as PBS, passes through the labeled secondary antibody carrying area. In this step, setting are made so that the labeled secondary antibody and the antigen are bound and the labeled secondary antibody bound to the antigen is released from the carrier and reaches the subsequent detection area. Then, the antigen bound to the labeled secondary antibody which arrives at the detection area binds to a primary antibody immobilized on the detection area to form a sandwich structure, which is a sample for detection.

In the present invention, examination of the presence of an antigen and determination thereof are performed using a sample prepared as described above by detecting magnetic signals emitted from magnetic beads constituting a sandwich structure immobilized on a detection area. Accordingly, the method of detecting magnetic signals is described below.

A usual commercially available magnetic sensor may be used as the magnetic sensor for detecting magnetic signals in the present invention. Examples thereof include Hall elements, semiconductor MR elements (SMR elements) and GMR (giant magnetoresistance) elements. These magnetic sensors may be used alone or a plurality of sensors may be provided depending on the number of analytes and the detection method. In some cases, elements may be made smaller and arrayed to perform measurement. Sensor chips to be employed are selected based on the sensitivity to analytes, the cost of the chip, and reliability, stability and the like in the measurement. Of such elements, semiconductor SMR elements are preferred in view of the price and the detection sensitivity.

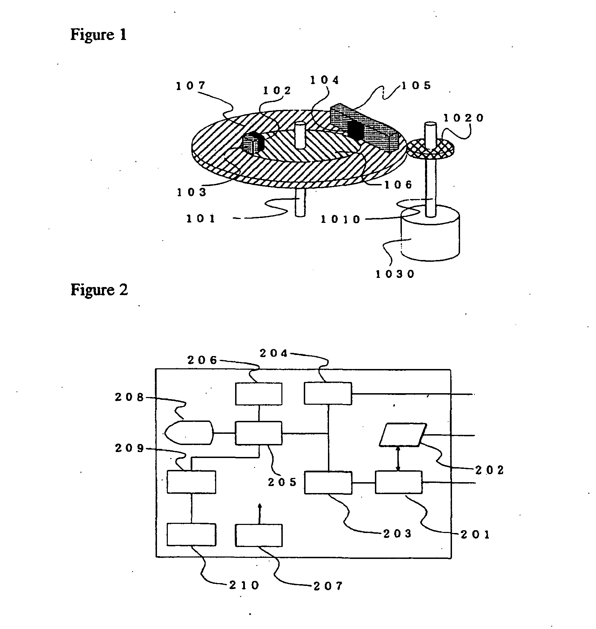

Accordingly, a method of detecting magnetic signals using a semiconductor SMR element (hereinafter magnetoresistive sensor) is described below.
Specifically, measurement is performed using a magnetic measuring instrument shown in Figure 1 and a signal processor shown in Figure 2. Herein, Figure 1 is a schematic view illustrating an embodiment of a magnetic signal detection system in the present invention. Reference numeral 101 denotes a two dimensional rotation center and reference numeral 102 denotes a magnetic field generator which produces a magnetic field in the normal direction of the rotation center 101. Reference numeral 103 denotes a magnetoresistive sensor positioned perpendicularly to the magnetic field produced by the magnetic field generator 102. Reference numeral 104 denotes a sample base for arranging the magnetoresistive sensor 103 and a sample 105 whose magnetism is measured in parallel. Reference numeral 106 denotes a stationary table equipped with the two dimensional rotation center 101, on which the magnetic field generator 102 and the magnetoresistive sensor 103 are fixed. Reference numeral 107 is a rotary table on which the sample base 104 is fixed and which is rotatable with the two dimensional rotation center 101 being the center. The rotary table 107 can move two-dimensionally and concentrically relative to the stationary table 106 by means of a driving function 1030, a drive rotation center 1010 and a drive transfer function 1020 with the rotation center 101 as the center.

The magnetic measuring instrument used in the present invention is described in more detail. A magnetoresistive element, BS05 made by Murata Manufacturing Co., Ltd., Japan, provided on a stationary table made of SUS304 is used as a magnetoresistive sensor housing a permanent magnet. A plastic sample base is settled on an aluminum rotary table, and two-dimensional, concentric relative movement between the sample and the magnetoresistive sensor is achieved. A negative feedback two-stage amplifier using two Operation Amplifiers LF-356M made by National Semiconductor Corporation, USA is employed as the amplifier. The circuit constant is determined so that the voltage amplification is 50,000 to 5,000,000 times.
A speed control motor which is M315-401 made by ORIENTAL MOTOR Co., Ltd., Japan, equipped with Gearhead 3GN15K made by the same company and a timing belt are used as a driving function and a drive transfer function. The optimal rotation number is determined in view of the sensitivity of the magnetoresistive sensor, the voltage amplification and generation of noise.

The signal obtained from the magnetic measuring instrument as described above is processed by the signal processor described below to detect the presence or absence and the amount of an antigen.
Figure 2 illustrates the result of processing of a signal detection system in the present invention, which is a block diagram describing a step for controlling a driving function capable of rotating a sample two-dimensionally and concentrically with the selected rotation center as the center relative to a signal converter for converting a magnetic measurement signal obtained from a magnetoresistive sensor to a processable form, a magnetic field generator and a magnetoresistive sensor.

An amplifier 201 amplifies output signals from the magnetoresistive sensor. Current amplification or voltage amplification is employed depending on the kind of the sensor. A position detection means 202 is not essential, but is preferably provided in the case where an approach of averaging processing is employed in the signal processing, or in the case where a signal is inputted while synchronizing with the position of the sample for improving the signal/noise ratio. Commonly used means such as a magnetic sensor which detects a magnet installed on the two dimensional rotation center or the rotary table, an optical sensor which detects a maker installed on the two dimensional rotation center or the rotary table, and a switch which detects a projection installed on the two dimensional rotation center or the rotary table may be used as a position detection means.

An analogue/digital converter 203 is a means for converting an analogue signal amplified in the amplifier 201 to a processable and storable digital signal, and a usual circuit may be used. A drive control function 204 controls the driving function, controls the rotation speed of the two dimensional rotation center and the rotary table, and works together with the position detection means 202 to finely control the rotation speed.

The central processing unit 205 executes computation of the digitalized signal, storing, transmission of the data to display means and communication with an external device. A communication means 206 for communicating with an external device transmits the measurement result obtained to a computer, a portable storage medium, a printer or the like. A power 207 supplies power to the entire signal processor. A display means 208 visualizes the processed signal, and a liquid crystal display, a plasma display, a light emitting diode, a neon tube, a Braun tube or the like is used. In the present invention, DS-4264, a digital oscilloscope made by Iwatsu Test Instruments Corporation, Japan, is used.
A storage medium 209 temporarily stores signals during processing or temporarily stores processed results. Preferably, a semiconductor storage element is used. A battery 210 for back up of the accumulated data is employed as required in the storage medium.
The method of detecting magnetic signals which may be used in the present invention has been described above.
Although a method of detecting an antigen according to an embodiment in which a sample rotates around a magnetoresistive sensor has been shown in the above description, a method of detecting an antigen according to an embodiment in which a sample reciprocates in the vicinity of a magnetic sensor may also be used. Examples

In the following, the present invention is described with reference to Examples, but the present invention is not limited to these Examples.

### [Example 1]

### [Labeling of anti-pneumococcal secondary antibody with magnetic beads via PEG chain]

In the first step of labeling an anti-pneumococcal secondary antibody with magnetic beads via a PEG chain, a PEG chain was attached to the antibody using Biotin-PEG-CO₂-NHS (MW3400 available from Shearwater Polymers Inc., USA) as described below.

6.8 mg of Biotin-PEG-CO₂-NHS reagent (MW3400 available from Shearwater Polymers Inc., USA) was measured and dissolved in 100 µl of distilled water to prepare a 20 mM aqueous solution thereof.
108 µl of a purified fraction of an anti-C-polysaccharide antibody (rabbit polyclonal antibody available from Statens Serum Institut, Denmark) through a protein G column (available from Pharmacia, Sweden) which was subjected to desalting and buffer exchange into PBS (antibody concentration 9.26 mg/ml) was mixed with 3.3 µl of the 20 mM Biotin-PEG-CO₂-NHS aqueous solution previously prepared. The mixture was allowed to react at room temperature for 2 hours.
The above reaction mixture was concentrated on a centrifugal ultrafiltration membrane (cut off molecular weight: 30,000) available from Millipore Corporation, USA at a rotational speed of 7500 rpm for 10 minutes. To the concentrate was further added 3 ml of PBS, and the mixture was concentrated again on the same ultrafiltration membrane under the same conditions. The procedure of adding 3 ml of PBS and concentrating under the same conditions was repeated twice to give a purified PEG-biotinylated antibody solution from which unreacted Biotin-PEG-CO₂-NHS was removed.
The degree of biotin labeling per molecule of the obtained PEG-biotinylated antibody solution was determined using a biotin determination reagent in an EZ-link Sulfo-NHS-Biotinylation reagent kit (available from Pierce Biotechnology, Inc., USA). As a result, the number of labels biotin per antibody molecule was 2.8 molecules (IgG concentration: 3 mg/ml).

Then, 100 µl of a 1% PBS solution of Dynabeads M-270 streptavidin (available from Dynal Biotech ASA, Norway, diameter 2.8 µm) was measured in an Eppendorf tube, and thereto was added 33 µl of the aforementioned PEG-biotinylated antibody solution. The total volume was adjusted to 500 µl with 367 µl of PBS, and the mixture was allowed to react with stirring at room temperature for 1 hour. Only the magnetic bead labeled secondary antibody was recovered from the magnetic bead labeled secondary antibody solution obtained in the reaction using a stationary magnet available from Dynal Biotech ASA, Norway, and the supernatant was removed. 1 ml of PBS was further added thereto and only the secondary antibody labeled with magnetic beads via a PEG chain was recovered and washed by a similar procedure. The resultant was finally dissolved in a 1% BSA/PBS solution so that the concentration of the prepared beads was 0.5%.
The secondary antibody labeled with magnetic beads via a PEG chain prepared as above was subjected to the immunoassay test of Example 2.

For comparative experiment, beads in which the same magnetic beads (Dynabeads M-270 streptavidin) were coupled to the secondary antibody without PEG were prepared.
In the experiment, the secondary antibody was biotinylated using an EZ-link Sulfo-NHS-Biotinylation kit reagent available from Pierce Biotechnology, Inc., USA in accordance with the instruction in the specification. Specifically, 20 µl of a 20 mg/ml Sulfo-NHS-Biotin aqueous solution was added to 1 ml of the previously used purified fraction of an anti-C-polysaccharide antibody (rabbit polyclonal antibody available from Statens Serum Institut, Denmark) through a protein G column (available from Pharmacia, Sweden) which was subjected to desalting and buffer exchange into PBS (antibody concentration 9.26 mg/ml), and the mixture was allowed to react at room temperature for 30 minutes. The resulting reaction mixture was subjected to desalting and buffer exchange using a D-salt dextran desalting column included in the kit with 3 times the bed volume of a PBS solvent.
The degree of biotin labeling per molecule of the obtained biotinylated antibody was determined by a biotin determination reagent included in the kit. As a result, the number of labels biotin per antibody molecule was 3.5 molecules (IgG concentration: 4 mg/ml).

Then, 100 µl of a 1% PBS solution of Dynabeads M-270 streptavidin (available from Dynal Biotech ASA, Norway, diameter 2.8 µm) was measured in an Eppendorf tube, and thereto was added 25 µl of the biotinylated antibody solution. The total volume was adjusted to 500 µl with 375 µl of PBS, and the mixture was allowed to react with stirring at room temperature for 1 hour. Only the magnetic bead labeled secondary antibody was recovered from the magnetic bead labeled secondary antibody solution obtained in the reaction using a stationary magnet available from Dynal Biotech ASA, Norway, and the supernatant was removed.
1 ml of PBS was further added thereto and only the magnetic bead labeled secondary antibody was recovered and washed by a similar procedure. The resultant was finally dissolved in a 1% BSA/PBS solution so that the concentration of the prepared beads was 0.5% to give a magnetic bead labeled secondary antibody reagent without a PEG chain for comparative experiment.

### [Example 2]

### [C-polysaccharide immunoassay with magnetic bead labeled secondary antibody and signal detection by magnetoresistive sensor]

50 µl of an anti-C-polysaccharide antibody (an antibody fraction of a rabbit polyclonal antibody purified through a protein G column, available from Statens Serum Institut, Denmark) dissolved in a 0.1 M sodium phosphate buffer (pH7) in a concentration of 10 µg/ml was spotted on a polystyrene plate (area: 1 cm square at the tip, 1 mm thick). The mixture was allowed to react at room temperature for 1 hour in a humidified box.

The surface of the plate was washed with distilled water, and 50 µl of a 0.1M sodium phosphate buffer (pH7) solution in 1% bovine serum albumin was spotted thereon, and reaction was performed at room temperature for 1 hour in a humidified box.

The surface of the plate was washed with distilled water and air-dried with drafting for 10 minutes. Then, 20 µl of a diluted normal saline solution of a C-polysaccharide antigen having a concentration of 10(ng/ml), 100(ng/ml) or 1000(ng/ml) was spotted on a primary antibody fixed area, and reaction was performed at room temperature for 10 minutes. The surface was washed with distilled water again and water on the surface was wiped with a paper pad.

10 µl each of 0.5% solutions of the magnetic bead labeled secondary antibody prepared according to the two methods in Example 1 was then spotted on the surface of the plate where the antigen was immobilized, and reaction was performed at room temperature for 10 minutes.
The surface of the plate after completion of the reaction was washed with distilled water so that the attached beads do not come off. The bonding state of the beads on the surface after air drying was observed and evaluated using a CCD camera at a magnification of 10 in the presence of scattered light in a diagonal direction. At the same time, magnetic signals derived from the magnetic beads on the surface of the plate were measured using a magnetoresistive sensor to compare the intensity of the signals. The measurement results are shown in Table 1 and Table 2.

The intensity of the magnetic signal was measured using the magnetic measuring instrument and the signal processor shown in Figure 1 and Figure 2, with driving at 50 RPM and setting the voltage amplification at 100,000 times.

**[Table 1]**

| Beads used | Conditions of labeling of secondary antibody with magnetic beads | C-polysaccharide concentration | | | |
|---|---|---|---|---|---|
| | | 1000 ng/ml | 100 ng/ml | 10 ng/ml | Negative sample |
| Dynabeads M-270 Streptavidin | Secondary antibody labeled with magnetic beads via biotinylated PEG chain | ○ | ○ | ○ | x |
| | Secondary antibody labeled with magnetic beads only via biotin (no PEG chain) | O | × | × | × |

| | | | | | |
|---|---|---|---|---|---|
| In the table, "O" means that magnetic beads were observed in the CCD camera observation. "×" means that magnetic beads were not observed in the CCD camera observation. | | | | | |

**[Table 2]**

| Beads used | Conditions of labeling of secondary antibody with magnetic beads | C-polysaccharide concentration | | | |
|---|---|---|---|---|---|
| | | 1000 ng/ml | 100 ng/ml | 10 ng/ml | Negative sample |
| Dynabeads M-270 Streptavidin | Secondary antibody labeled with magnetic beads via biotinylated PEG chain | 5V | 3V | 0.7 V | below detection limit |
| | Secondary antibody labeled with magnetic beads only via biotin (no PEG chain) | 1V | below detection limit | below detection limit | below detection limit |

### [Example 3]

### [Labeling of anti-Mycoplasma purified protein secondary antibody with magnetic beads via PEG chain]

In the first step of labeling an anti-Mycoplasma purified protein secondary antibody with magnetic beads via a PEG chain, a PEG chain was attached to the antibody using Biotin-PEG-CO₂-NHS (MW3400 available from Shearwater Polymers Inc., USA). 2.9 mg of a Biotin-PEG-CO₂-NHS reagent (MW3400 available from Shearwater Polymers Inc., USA) was measured and dissolved in 200 µl of distilled water to prepare a 4.26 mM aqueous solution thereof.
1.5 ml of anti-Mycoplasma antibody AMMP-1 disclosed in European Patent No. 1104772 which was subjected to desalting and buffer exchange into PBS (antibody concentration 6.99 mg/ml) and 49.2 µl of the 4.26 mM aqueous solution of Biotin-PEG-CO₂-NHS prepared above were mixed and allowed to react at room temperature for 4 hours.
The above reaction mixture was concentrated on a centrifugal ultrafiltration membrane available from Millipore Corporation, USA (cut off molecular weight: 30,000) at a rotational speed of 7500 rpm for 10 minutes. To the concentrate was further added 3 ml of PBS and the mixture was concentrated again on the same ultrafiltration membrane under the same conditions. The procedure of adding 3 ml of PBS and concentrating under the same conditions was repeated twice to give a purified PEG-biotinylated antibody solution from which unreacted Biotin-PEG-CO₂-NHS was removed.
The degree of biotin labeling per molecule of the obtained PEG-biotinylated antibody solution was determined using a biotin determination reagent in an EZ-link Sulfo-NHS-Biotinylation reagent kit (available from Pierce Biotechnology, Inc., USA). As a result, the number of labels biotin per antibody molecule was 1.3 molecules (IgG concentration: 10.5 mg/ml).

Then, 100 µl of a 1% PBS solution of Dynabeads MyOne streptavidin (available from Dynal Biotech ASA, Norway, diameter 1.0 µm) was measured in an Eppendorf tube, and thereto was added 40 µl of the aforementioned PEG-biotinylated antibody solution. The total volume was adjusted to 500 µl with 460 µl of PBS, and the mixture was allowed to react with stirring at room temperature for 4 hours. Only the magnetic bead labeled secondary antibody was recovered from the magnetic bead labeled secondary antibody solution obtained in the reaction using a stationary magnet available from Dynal Biotech ASA, Norway, and the supernatant was removed.
1 ml of PBS was further added thereto and only the secondary antibody labeled with magnetic beads via a PEG chain was recovered and washed by a similar procedure. The resultant was finally dissolved in a 1% BSA/PBS solution so that the concentration of the prepared beads was 0.05%.
The secondary antibody labeled with magnetic beads via a PEG chain prepared as above (hereinafter magnetic bead-labeled, PEG-attached secondary antibody 1) was subjected to the immunoassay test of Example 4.

Further, beads in which the same magnetic beads (Dynabeads Myone streptavidin) were coupled to the secondary antibody via PEG having a lower molecular weight were prepared.
Specifically, a substance obtained by introducing -SH into 250 µl of the anti-Mycoplasma antibody AMMP-1 (5.18 mg/ml) previously used by a method in a known literature was used (see Anal. Biochem. 132, 68-74), and 7.1 µl of 53.3 mM Biotin-PEG-Maleimide (available from Pierce Biotechnology, Inc., USA) was added thereto. The mixture was allowed to react at room temperature for 2 hours. The resulting reaction mixture was concentrated on a centrifugal ultrafiltration membrane available from Millipore Corporation, USA (cut off molecular weight: 30,000) at a rotational speed of 7500 rpm for 10 minutes. To the concentrate was further added 3 ml of PBS, and desalting and washing were repeated three times under the same conditions to give 1.4 mg/ml low molecular weight PEG-biotinylated antibody solution. The degree of biotin labeling per molecule of the obtained biotinylated antibody was determined by a biotin determination reagent included in the kit. As a result, the number of labels biotin per antibody molecule was 6.8 molecules.

Then, 100 µl of a 1% PBS solution of Dynabeads Myone streptavidin (available from Dynal Biotech ASA, Norway, diameter 1.0 µm) was measured in an Eppendorf tube, and thereto was added 306.6 µl of the aforementioned low molecular weight PEG-biotinylated antibody solution. The total volume was adjusted to 500 µl with 193.4 µl of PBS, and the mixture was allowed to react with stirring at room temperature for 4 hours. Only the magnetic bead labeled secondary antibody was recovered from the magnetic bead labeled secondary antibody solution obtained in the reaction using a stationary magnet available from Dynal Biotech ASA, Norway, and the supernatant was removed.
1 ml of PBS was further added thereto and only the magnetic bead labeled secondary antibody was recovered and washed by a similar procedure. The resultant was finally dissolved in a 1% BSA/PBS solution so that the concentration of the prepared beads was 0.05% to give a low molecular weight PEG chain magnetic bead labeled secondary antibody reagent (hereinafter magnetic bead-labeled, PEG-attached secondary antibody 2).

For comparative experiment, beads in which the same magnetic beads (Dynabeads Myone streptavidin) were coupled to the secondary antibody without PEG were prepared.
In the experiment, the secondary antibody was biotinylated using an EZ-link Sulfo-NHS-Biotinylation kit reagent available from Pierce Biotechnology, Inc., USA in accordance with the instruction in the specification. Specifically, 4.5 µl of a 14.0 mM Sulfo-NHS-Biotin aqueous solution was added to 200 µl of the previously used anti-Mycoplasma antibody AMMP-1 disclosed in European Patent No. 1104772 which was subjected to desalting and buffer exchange into PBS (antibody concentration 6.99 mg/ml), and the mixture was allowed to react at room temperature for 3 hours. The resulting reaction mixture was subjected to desalting and buffer exchange using a D-salt dextran desalting column included in the kit with 3 times the bed volume of a PBS solvent to give a biotinylated antibody solution.
The degree of biotin labeling per molecule of the obtained biotinylated antibody was determined by a biotin determination reagent included in the kit. As a result, the number of labels biotin per antibody molecule was 1.6 molecules (IgG concentration: 2.05 mg/ml).

Then, 100 µl of a 1% PBS solution of Dynabeads MyOne streptavidin (available from Dynal Biotech ASA, Norway, diameter 1.0 µm) was measured in an Eppendorf tube, and thereto was added 204.8 µl of the biotinylated antibody solution. The total volume was increased to 500 µl with 295.2 µl of PBS, and the mixture was allowed to react with stirring at room temperature for 4 hours. Only the magnetic bead labeled secondary antibody was recovered from the magnetic bead labeled secondary antibody solution obtained in the reaction using a stationary magnet available from Dynal Biotech ASA, Norway, and the supernatant was removed.
1 ml of PBS was further added thereto and only the magnetic bead labeled secondary antibody was recovered and washed by a similar procedure. The resultant was finally dissolved in a 1% BSA/PBS solution so that the concentration of the prepared beads was 0.05% to give a magnetic bead labeled secondary antibody reagent without a PEG chain for comparative experiment (hereinafter magnetic bead labeled secondary antibody 3).

### [Example 4]

### [Mycoplasma purified protein immunoassay with magnetic bead labeled secondary antibody and signal detection by magnetoresistive sensor]

50 µl of anti-Mycoplasma antibody AMMP-3 disclosed in European Patent No. 1104772 (derived from MPRB-3 in the same specification) dissolved in 0.1M sodium phosphate buffer (pH7) in a concentration of 10 µg/ml was spotted on a polystyrene plate (area: 1 cm square at the tip, 1 mm thick). Reaction was performed at room temperature for 1 hour in a humidified box.
The surface of the plate was washed with distilled water, and 50 µl of 0.1M sodium phosphate buffer (pH7) solution containing 1% bovine serum albumin was spotted thereon, and reaction was performed at room temperature for 1 hour in a humidified box.
The surface of the plate was washed with distilled water and air-dried with drafting for 10 minutes. Then, 20 µl of a diluted normal saline solution of a purified antigen (ribosomal protein L7/L12 of Mycoplasma pneumoniae) with a concentration of 10 (ng/ml), 100 (ng/ml) or 1000(ng/ml) was spotted on a primary antibody fixed area, and reaction was performed at room temperature for 10 minutes. The surface was washed with distilled water again and water on the surface was wiped with a paper pad.

5 µl each of 0.05% solutions of the magnetic bead labeled secondary antibody prepared by the three methods in Example 3 was then spotted on the surface of the plate where the antigen was immobilized, and reaction was performed at room temperature for 10 minutes.
The surface of the plate after completion of the reaction was washed with distilled water so that the attached beads do not come off. The bonding state of beads on the surface after air drying was observed and evaluated using a CCD camera at a magnification of 10 in the presence of scattered light in a diagonal direction. At the same time, magnetic signals derived from magnetic beads on the surface of the plate were measured using a magnetoresistive sensor to compare the intensity of the signals. The measurement results are shown in Table 3 and Table 4.
The intensity of the magnetic signal was measured as in Example 2 using the magnetic measuring instrument and the signal processor shown in Figure 1 and Figure 2, with driving at 50 RPM and setting the voltage amplification at 100,000 times.

**[Table 3]**

| Beads used | Conditions of labeling of secondary antibody with magnetic beads | Purified antigen concentration antigen: ribosomal protein L7/L12 of Mycoplasma pneumoniae | | | |
|---|---|---|---|---|---|
| | | 100 ng/ml | 10 ng/ml | 1 ng/ml | Negative sample |
| Dynabeads MyOne Streptavidin | Magnetic bead-labeled, PEG-attached secondary antibody 1 | ○ | ○ | ○ | × |
| | Magnetic bead-labeled, PEG-attached secondary antibody 2 | ○ | ○ | × | × |
| | Magnetic bead labeled secondary antibody 3 (no PEG) | ○ | ○ | × | × |

| | | | | | |
|---|---|---|---|---|---|
| In the table, "○" means that magnetic beads were observed in the CCD camera observation. "×" means that magnetic beads were not observed in the CCD camera observation. | | | | | |

**[Table 4]**

| Beads used | Conditions of labeling of secondary antibody with magnetic beads | Purified antigen concentration antigen: ribosomal protein L7/L12 of Mycoplasma pneumoniae | | | |
|---|---|---|---|---|---|
| | | 100 ng/ml | 10 ng/ml | 1 ng/ml | Negative sample |
| Dynabeads MyOne Streptavidin | Magnetic bead-labeled, PEG-attached secondary antibody 1 | 601 mV | 83 mV | 52 mV | below detection limit |
| | Magnetic bead-labeled, PEG-attached secondary antibody 2 | 337 mV | 55 mV | below detection limit | below detection limit |
| | Magnetic bead labeled secondary antibody 3 (no PEG) | 462 mV | 57 mV | below detection limit | below detection limit |

### Industrial Applicability

The present invention provides a novel detecting technique of analytes with high sensitivity which can be applied to examination of the presence of various analytes, the qualification and the determination thereof. In particular, the present invention provides such a technique which can be suitably applied to the field of medical diagnosis and test agents including tests on antigens contained in blood, various body fluids, wipe liquids and the like using immunoassay.

## Claims

1. A labeled specific binding material comprising a substance capable of specifically binding to an analyte, a spacer and magnetic beads having a particle size of 0.5 to 10 µm, wherein the specific binding substance is coupled to the magnetic beads via the spacer, and
wherein the spacer is polyalkylene glycol having 50 to 500 repeat units.

2. The labeled specific binding material according to claim 1, wherein the polyalkylene glycol is polyethylene glycol.

3. The labeled specific binding material according to any one of claims 1 to 2 , wherein the spacer is bonded to the magnetic beads through an avidin/biotin complex.

4. The labeled specific binding material according to any one of claims 1 to 3 , wherein the analyte is an antigen and the substance capable of specifically binding to the analyte is an antibody.

5. A kit for detecting an analyte, comprising a labeled specific binding material according to any one of claims 1 to 4.

6. An in vitro method of detecting an analyte, comprising binding the analyte to the labeled specific binding material according to any one of claims 1 to 4 to form a conjugate, and detecting a magnetic signal from the conjugate to detect the analyte.

7. In vitro use of a labeled specific binding material according to any one of claims 1 to 4 for detecting an analyte.

8. Use of a labeled specific binding material according to any one of claims 1 to 4 for provision of a kit for detecting an analyte.

## Patentansprüche

1. Markiertes spezifisches Bindungsmaterial, umfassend eine Substanz, welche in der Lage ist, spezifisch an einen Analyten zu binden, einen Abstandshalter und magnetische Kügelchen mit einer Teilchengröße von 0,5 bis 10 µm, wobei die spezifische Bindungssubstanz mittels eines Abstandshalters an die magnetischen Kügelchen gebunden ist, und wobei der Abstandshalter ein Polyalkylenglykol mit 50 bis 500 Wiederholungseinheiten darstellt.

2. Markiertes spezifisches Bindungsmaterial nach Anspruch 1, wobei das Polyalkylenglykol ein Polyethylenglykol darstellt.

3. Markiertes spezifisches Bindungsmaterial nach einem der Ansprüche 1 oder 2, wobei der Abstandshalter an die magnetischen Kügelchen über einen Avidin/Biotin-Komplex gebunden ist.

4. Markiertes spezifisches Bindungsmaterial nach einem der Ansprüche 1 bis 3, wobei der Analyt ein Antigen darstellt, und wobei die Substanz, welche in der Lage ist, spezifisch an einen Analyten zu binden, einen Antikörper darstellt.

5. Kit zum Nachweis eines Analyten, umfassend ein markiertes spezifisches Bindungsmaterial nach einem der Ansprüche 1 bis 4.

6. In vitro Verfahren zum Nachweis eines Analyten, umfassend das Binden des Analyten an das markierte spezifische Bindungsmaterial nach einem der Ansprüche 1 bis 4, um ein Konjugat zu bilden, und den Nachweis eines magnetischen Signals aus dem Konjugat zum Nachweis eines Analyten.

7. In vitro Verwendung eines markierten spezifischen Bindungsmaterials nach einem der Ansprüche 1 bis 4 zum Nachweis eines Analyten.

8. Verwendung eines markierten spezifischen Bindungsmaterials nach einem der Ansprüche 1 bis 4 zur Bereitstellung eines Kits zum Nachweis eines Analyten.

## Revendications

1. Matière marquée de fixation spécifique, comprenant une substance apte à se fixer spécifiquement à un analyte, un espaceur et des perles magnétiques ayant une dimension de particule de 0,5 à 10 µm, la surface spécifique de fixation étant couplée aux perles magnétiques par l'intermédiaire de l'espaceur et l'espaceur étant un polyalcylèneglycol ayant de 50 à 500 motifs de récurrence.

2. Matière marquée de fixation spécifique suivant la revendication 1, dans laquelle le polyalcylèneglycol est le polyéthylèneglycol.

3. Matière marquée de fixation spécifique suivant la revendication 1 ou 2, dans laquelle l'espaceur est fixé aux perles magnétiques par un complexe avidine/biotine.

4. Matière marquée de fixation spécifique suivant l'une quelconques des revendications 1 à 3, dans laquelle l'analyte est un antigène et la substance apte à se fixer spécifiquement à un analyte est une anticorps.

5. Trousse de détection d'un analyte comprenant une matière marquée de fixation spécifique suivant l'une quelconques des revendications 1 à 4.

6. Procédé in vitro de détection d'un analyte, comprenant la fixation de l'analyte à la matière marquée de fixation spécifique suivant l'une quelconque des revendications 1 à 4 pour former un conjugué et la détection d'un signal magnétique à partir du conjugué pour détecter l'analyte.

7. Utilisation in vitro d'une matière marquée de fixation spécifique suivant l'une quelconque des revendications 1 à 4 pour détecter un analyte.

8. Utilisation d'une matière marquée de fixation spécifique suivant l'une quelconque des revendications 1 à 4 pour procurer une trousse de détection d'un analyte.
